# EUROPEAN PATENT APPLICATION

(11) **EP 3 028 705 A1**
(43) Date of publication of application: **08.06.2016**
(21) Application number: 16151565.5
(22) Date of filing: 06.09.2007
(51) Int. Cl.: A61K 31/54, A61K 31/541, A61K 31/00, A61K 9/00, A61P 35/00

(54) **TREATING BONE CANCER**

(30) Priority: 07.09.2006 US 842657 P
(62) Divisional of application: 07825069.3
(71) Applicant: ED. GEISTLICH SÖHNE AG FÜR CHEMISCHE INDUSTRIE, 6110 Wolhusen (CH)
(72) Inventor: PFIRRMANN, Rolf, W., CH-6353 Weggis (CH)
(74) Representative: Matthews, Derek Peter

(57) **Abstract**

A gel containing a tumor growth-inhibiting methylol transfer agent for use in a method of treating, at least partially preventing, inhibiting or reducing growth of a bone tumor in a subject, said method including at least partially removing a bone tumor from a subject and contacting an area of bone adjacent to where the tumor was at least partially removed with said gel. The tumor growth-inhibiting methylol transfer agent preferably is taurolidine otr taurultam.

## Description

### BACKGROUND OF THE INVENTION

### Field of the Invention

This invention concerns the field of treating bone cancers.

### Description of the Background Art

Cancerous bone tumors refer to malignant abnormal growths found in bone, including primary tumors of bone, such as osteosarcoma (or osteoma). Bone tumors also may include secondary, or metastatic tumors found in bone.

Malignant primary bone tumors include osteosarcoma, chondrosarcoma, Ewing's sarcoma, and other sarcoma types. Multiple myeloma is a hematologic cancer which also frequently presents as one or more bone tumors.

Secondary bone tumors include metastatic tumors which have spread from other organs, such as the breast, lung, and prostate. Metastatic tumors more frequently involve the axial skeleton than the appendicular skeleton. Tumors which originate in the soft tissues may also secondarily involve bones through direct invasion.

Osteosarcoma is the most common type of malignant bone cancer, accounting for 35% of primary bone malignancies. There is a tendency toward the metaphyseal region of tubular long bones. 50% of cases occur around the knee. It is a malignant connective (soft) tissue tumor whose neoplastic cells present osteoblastic differentiation and form tumoral bone.

Osteosarcoma is the 6th leading cancer in children under age 15. Osteosarcoma affects 400 children under age 20 and 500 adults (most between the ages of 15-30) every year in the USA. Approximately 1/3 of the 900 will die each year, or about 300 a year. A second peak in incidence occurs in the elderly, usually associated with an underlying bone pathology such as Paget's disease, medullary infarct, or prior irradiation. Although about 90% of patients are able to have limb-salvage surgery, complications, such as infection, prosthetic loosening and non-union, or local tumor recurrence may cause the need for further surgery or amputation. The tumor may be localized at the end of the long bones. Most often it affects the upper end of tibia or humerus, or lower end of femur. The tumor is solid, hard, irregular ("fir-tree" or "sun-burst" appearance on X-ray examination) due to the tumor spicules of calcified bone radiating in right angles. These right angles form what is known as Codman's triangle. Surrounding tissues may be infiltrated.

Osteosarcoma is the most common bone tumor in dogs and typically afflicts middle-age large and giant breed dogs such as Irish Wolfhounds, Greyhounds, German Shepherds, Rottweilers, and Great Danes. It has a ten times greater incidence in dogs than humans. A hereditary base has been shown in St. Bernard dogs. Spayed/neutered dogs have twice the risk of intact ones to develop osteosarcoma.

There remains a need in the art for methods of treating bone cancers.

### SUMMARY OF THE INVENTION

A gel containing a tumour growth-inhibiting methylol transfer agent for use in the treatment, at least partial prevention, inhibition or reduction of the growth of a bone tumour in a subject, said use comprising at least partially removing a bone tumour from a subject and contacting an area of bone adjacent to where the tumour was at least partially removed with said gel.

### DETAILED DESCRIPTION OF THE INVENTION

The present invention treats bone cancers, such as osteosarcomas, by administering to a subject a gel containing a methylol transfer agent such as taurolidine, taurultam, a mixture thereof or an equilibrium thereof. In preferred embodiments, the gel is resorbable. It is particularly preferred that the gel be an aqueous gel comprised of or formed from cross-linked fibrous protein.

According to one embodiment, the cancerous tumor is a sarcoma, such as osteosarcoma, chondrosarcoma, or Ewing's sarcoma.

According to another embodiment, the tumor is a metastasis of a tumor or cancer from tissue outside other than bone.

Taurolidine acts by transferring three methylol groups at the site of action, taurultam being an intermediate metabolite which itself transfers a single methylol group with liberation of the very well tolerated compound taurinamide. Thus, the two compounds act by essentially the same mechanism. In solution, taurolidine and taurultam are in equilibrium.

By "methylol transfer agent," is meant a compound which contains or is capable of producing a methylol molecule under physiological conditions. A methylol-containing compound is characterized as having a R-CH₂-OH group in which R is an alkyl, aryl or hetero group. The invention also includes the use of compounds capable of producing or being converted into a compound containing a R-CH₂-OH structure.

Methylol transfer agents include methylol-containing compounds such as taurolidine and taurultam, and their derivatives. The compounds taurolidine and taurultam are disclosed in U.S. Patent No. 5,210,083. Other suitable methylol-containing compounds include taurinamide derivatives and urea derivatives. Examples of derivatives of taurolidine, taurultam, taurinamide and urea useful in the present invention can be found in WO 01/39763A2. Particularly preferred methylol transfer agents for utilization in accordance with the present invention are taurolidine, taurultam, mixtures and/or equilibriums thereof, biologically active derivatives thereof and mixtures thereof.

Alternatively, the compound is a taurinamide derivative, or a urea derivative. Examples of derivatives of taurolidine, taurultam, taurinamide and urea useful in the present invention can be found in WO 01/39763A2.

Other methylol-containing compounds suitable for inducing apoptotic death of cancer cells include but are not limited to 1,3,-dimethylol-5,5-dimethylhydantoin, hexamethylene tetramine, or noxythiolin. By derivative of taurolidine or taurultam is meant a sulfonamide compound which possesses at least 10% of the anti-neoplastic activity of taurolidine or taurultam, respectively. A sulfonamide compound is one having a R₂N-SO₂R' formula. Derivatives of the compounds described herein may differ structurally from a reference compound, e.g., taurolidine or taurultam, but preferably retain at least 50% of the biological activity, e.g., induction of apoptotic cell death, of the reference compound. Preferably, a derivative has at least 75%, 85%, 95%, 99% or 100% of the biological activity of the reference compound. In some cases, the biological activity of the derivative may exceed the level of activity of the reference compound. Derivatives may also possess characteristics or activities not possessed by the reference compound. For example, a derivative may have reduced toxicity, prolonged clinical half-life, or the like.

One embodiment of the invention provides a a resorbable, aqueous gel, the aqueous phase of which contains a water-soluble methylol transfer agent, and which treats, at least partially prevents, inhibits or reduces growth of cancer or tumor cells in bone, or in a cavity in bone, when the gel is placed in the cavity and allowed to remain therein until resorbed.

In a particularly preferred embodiment of the invention, however, the gel is relatively rapidly resorbed, for example over a period of a few weeks, advantageously 10 to 14 days, the methylol transfer agent being released primarily by the resorption process rather than by diffusion of the methylol transfer agent out of the gel.

The gel may be in the form of a shaped solid, such as a rod, for insertion into the cavity to be healed, thus possibly being 2 to 20 mm in diameter and 20 to 150 mm in length. In a particularly advantageous embodiment of the invention the gel is in the form of granules or a granulate. Using this form it is possible to fill completely cavities of any shape or size without the need for implants of specialized configurations.

The rapidly resorbable gel is preferably an aqueous gel comprising a cross-linked, water soluble fibrous protein, e.g. a scleroprotein such as tropocollagen or partially hydrolysed collagen, tropocollagen or elastin (including, in particular, gelatin). Elastin is the elastic fibrous protein of tendons and arteries. Collagen is the inelastic fibrous protein of skin, tendons and bones, and comprises strands of molecules of tropocollagen in a triple helix configuration. The tropocollagen can be liberated, for example from the skin of young mammals, by extraction with citrate buffer. The molecular weight of the partially hydrolysed collagen, tropocollagen or elastin is preferably in the range 100,000 to 350,000. When boiled in water, collagen yields the protein gelatin.

Preferably, the resorbable gel comprises or contains gelatin, even when other fibrous proteins are present. This ensures flexibility in the gel and avoids undue rigidity which in some cases could cause problems in inserting the gel into the cavity to be healed. Where a high degree of flexibility is advantageous, for example where the gel is in the form of a three dimensional shape such as a rod, the gel preferably contains 80% to 100% by weight of gelatin, advantageously substantially 100%. Where the gel is provided in the form of a granulate the degree of flexibility must however be balanced by a certain degree of firmness so as to allow proper granulation. In this case certain quantities of fibrous proteins other than gelatin may be used and the gelatin content may, for example, be in the range 60% to 80%, e.g. about 70% by weight of total protein, the remainder being tropocollagen or partially hydrolysed collagen, tropocollagen or elastin or, if desired fibres of unhydrolysed collagen or elastin. Naturally, such granulates can, if desired, contain higher quantities of gelatin, and 100% gelatin may be used, if sufficiently cross-linked.

The properties of gelatin may be influenced by its mode of manufacture. So called edible gelatin is made by the acid hydrolysis of skin collagen and it is found to give a pH of about 4.2 on dispersion in water. Bone gelatin, on the other hand, is often prepared by alkaline hydrolysis of bone collagen and on dispersion in water gives a less acidic pH e.g. about 6.0. We have found that on reaction with equal amounts of cross-linking agent, the less acid bone gelatin gives a firmer more resilient gel than does the more acidic edible gelatin. However, it may be preferable to neutralize the gelatin solution e.g. to about 7.0 prior to cross-linking, in order to make the gel more completely physiologically compatible.

The fibrous protein preferably comprises 5 to 30% by weight of the gel, advantageously about 10-20%, depending on the properties desired. The gel preferably remains solid at body temperatures but this is not essential. Thus, for example, when the gel is relatively hard, resorption is slower. This can be achieved by using more formaldehyde. Similarly, if faster resorption is desired, a softer gel may be appropriate. Harder gels may be preferable where granulates are required in that they are more readily mechanically granulated.

The gels can also contain other additives which desirably influence their physical and/or biochemical properties. One useful such additive is calcium phosphate which has the effect of improving the firmness of the gels. Furthermore, it is believed that the calcium phosphate may act to supply calcium to the bone by sustained release when the gel is in place in the cavity. Polysaccharides and polyvinyl-pyrrolidone, particularly of higher molecular weight, e.g. about 40,000, also may provide slower resorption.

In addition to methylol transfer agent, further medicaments, for example analgesic agents, which are soluble in the swelling water of the gels may be used. In addition, the swelling water can also contain other dissolved additives which promote healing of the wound and/or favorably influence the physical and biochemical properties of the gel. These are, for example, amino acids, sugar, polyhydric alcohols, common salt and others. Finally, the gels can also contain an X-ray contrast agent.

The preferred active substances are methylol transfer agents such as taurolidine, taurultam, mixtures thereof or equilibriums thereof. Where taurolidine and/or taurultam is used, its concentration in the aqueous solution absorbed in the gel is preferably 0.5% to 5% by weight, e.g. about 1 to 4%. Where the gel is in the form of a granulate, 2-4% by weight taurolidine and/or taurultam is preferred, more preferably, 4% taurolidine and/or taurultam by weight. Taurolidine is only about 2-3% soluble in water at room temperature, so that at higher concentrations, some material will be present as a suspension of, for example, fine taurolidine crystals.

A complex of elemental iodine and polyvinylpyrrolidone may also be included.

Cross-linking of the fibrous protein may be necessary to facilitate the cohesion of the gel and also serves to reduce immunological reactions to the "foreign" protein by reacting with free amino groups. The preferred cross-linking agent is a methylol transfer agent such as formaldehyde or a methylol transfer agent derived from formaldehyde, such as the taurolidine and/or taurultam. In general, using formaldehyde as the cross-linking agent, the percentage of bound formaldehyde in the gel relative to protein is preferably in the range 2.0 to 5.0, advantageously 2.3 to 4.0. Thus, for example, using 10% aqueous gelatin, it is convenient to add initially about 3.6% formaldehyde, the level of bound formaldehyde falling, after washing, to about 2.7%. However, if only about 2.7% formaldehyde is added initially, subsequent washing may often be dispensed with. In preferred embodiments, substantially no free formaldehyde is present in the final product. Furthermore, the invention allows cross-linked gels to be provided which employ no toxic cross-linking agents such as those used for cross linking some other polymeric materials. This means that there is no risk of residual amounts of toxic substances being present in the gels when they are placed in the bone cavity. In cases where the gels according to the invention are resorbable, they have the advantage that only one operation may be necessary in the treatment. Once the cavity has been filled with the gel the wound can be closed and should not need to be opened again.

Where the gel is cross-linked using formaldehyde, it is conveniently prepared by warming an appropriate quantity of the gel-forming protein in an aqueous solution of the methylol transfer agent, and any other desired components, to dissolve the protein and then adding formaldehyde, preferably in aqueous solution or in the form of a polymer of formaldehyde such as paraldehyde. Formalin, which is a 36-40% aqueous solution of formaldehyde, is especially convenient. Where gelatin is used, this may be for example edible gelatin or bone gelatin. As indicated above, bone gelatin generally gives a harder or firmer gel than edible gelatin. Generally, per 100 g of aqueous solution, containing e.g. 0.3-4.75 g of taurolidine and/or taurultam, 7.0-12.0 g, e.g. 10.0 g of gelatin will be used, optionally with 1-35, e.g. 25 g of dibasic calcium phosphate. The aqueous solution may contain, in addition to taurolidine and/or taurultam, such additives as gentamycin sulphate, chondroitin sulphate, and polyvinylpyrrolidone; finely ground bone powder may also be added if desired. Generally about 0.75-1.0 g of about 36% aqueous formaldehyde will be used in such a mixture. The solution is then poured into one or more preheated moulds, for example a length of tubing, and allowed to cool. After cooling and setting, the gel may, if required, be cut into suitably sized sections, and these may be granulated if desired by means of a conventional granulating machine or mincer. The granulate should generally be of average diameter in the range of about 0.1 -10 mm, preferably about 1 - 5 mm, more preferably about 1.5 - 5 mm, and advantageously about 1.5 - 5 mm to enable it readily to be filled into the cavity but not to be washed out by exudation. It is possible in some instances for the granulate to be of such fine grain that it can be used post-operatively, by being instilled into the cavity by means of a syringe via a drain. Particle sizes less than 0.5 mm are preferred (e.g. about 0.4 mm) for this purpose.

The gel may contain a small quantity of free formaldehyde after preparation, and this may be removed by washing until no further formaldehyde appears in the wash water; in order to avoid removing the methylol transfer agent at the same time, the washing is preferably effected with an aqueous solution of the methylol transfer agent. Testing using gas chromatography (GC-WLD or FID) can detect free formaldehyde down to 0.003%. As indicated above, the amount of formaldehyde added initially is greater than that finally bound in the gel, after washing. In general, 4% of formaldehyde (relative to protein) may be added initially to produce 2.7% bound formaldehyde. Where polyvinylpyrrolidone is added, the percentage of formaldehyde is preferably lower e.g. about 3%.

The gel may take 24 hours or more for solidification, but it is advantageous to leave the gel for a longer period than this before washing and (if desired) granulation. Thus, the gel may be left for at least 1 to 8 days, advantageously 4 to 7 days during which time its firmness is greatly improved due possibly to the continuance of cross-linking reactions within the gel. This procedure is particularly advantageous where the gel is to be provided in the form of a granulate as the increase in firmness improves the granulability of the gel.

As indicated above, cross-linking may be effected by methylol transfer agents such as taurolidine and/or taurultam. Thus, taurolidine may be incorporated at a level of about 4.75% into a solution of edible gelatin and left for several days. There may be a slight fall in the level of active taurolidine for example to about 3.7%, but cross-linking occurs to yield a gel of satisfactory firmness. No washing is required for the removal of excess formaldehyde.

It has been found useful in certain circumstances to reduce the water content of the gel material by partial drying. The material may for example be dried to reduce the water content by 60-80%, e.g., about 70% by weight.

Drying may be effected by laying strips or sheets of gel in an oven or warm air cabinet at a temperature slightly above ambient, such as 30-50, e.g. about 40°C. Vacuum drying may be used as an alternative. The degree of dehydration should be carefully monitored, as it is not intended that the material should be completely dehydrated. Drying of the material in this way has been found to have the advantage of increasing the firmness and granulability of the gels.

If the gel is left in the form of rods or other shapes, these may conveniently be sterile packed in suitable water- and air- impermeable packaging material, such as sealable polyvinyl chloride or polyethylene foil sterilized, for instance, by washing with 70% aqueous isopropanol. The foil may be backed with paper and/or aluminum foil to increase water impermeability. If all the previous steps are effected under sterile conditions, no further sterilization will be required. Otherwise sterilization may be effected using ethylene oxide or formaldehyde. Thus, for example, the gel may be left, e.g. for about 20 days, with about 100 ppm ethylene oxide. The ethylene oxide level falls around this period to about 1-2 ppm due to hydrolysis and is subsequently removed. Sterility may be more readily maintained if an inner and an outer envelope packaging foil is used, the inner envelope only being taken into the operating theatre.

One particularly useful method of treatment according to the invention is to mix the gel in sterile granular form with autologous spongiosa tissue obtained from a healthy bone of the same patient and/or an artificial or natural bone mineral such as Bio-Oss® (Geistlich AG). The iliac crest can provide small quantities of spongiosa tissue, while larger quantities can be obtained from the trochanter major and spina iliaca posterior. The mixture of gel to bone or bone mineral can be in any suitable ratio, such as 1:9, 1:4, 3:7, 2:3, 1:1, 3:2, 7:3, or 4:1. In this mode of use, the gel should be isotonic, in order to avoid osmotic effects on contact with the spongiosa tissue. The aqueous phase of the gel can thus be physiological saline or Ringer lactate solution. (0.22% lactic acid, 0.6% NaCl, 0.4% KCl, 0.4% CaCl₂ 6H₂O; neutralised with NaOH to be orange to phenol red indicator (pH 7.0), sterilised for 15 minutes at 12°C.) In general, the gels of the invention have colloid osmotic pressure compatible with the skin. The colloid osmotic pressure may be enhanced by incorporation of a low molecular weight polyvinylpyrrolidone (PVP), e.g. in the molecular weight range 8000-12,000 daltons, for example about 11,000 daltons. In that the salts in such solutions may affect the setting of the gel on cooling, they are preferably introduced after the gel has set, by including them in the washing solution used to remove formaldehyde. In order to accelerate incorporation of the salts into the gel, the concentrations of the salts in such wash water may be hypertonic and their uptake into the gel may be monitored until isotonicity is achieved. However, uptake is quite rapid from isotonic solutions.

The invention is applicable to treating mammals, including human and canine subjects. When treating dogs, it is preferred that the gel containing taurolidine and/or taurultam be free of PVP, because some dogs are extremely sensitive to PVP. In PVP-free compositions, citric acid (e.g., 0.01-0.05 wt. %) can be substituted for the PVP.

Depending on the particular case, a drain tube leading from the bone cavity to outside the subject can be installed for drainage of the area, after which the cavity is surgically closed. When healing is taking place rapidly, there may be a healthy exudation of fluid.

In particularly preferred embodiments, the gels contained 4% taurolidine and/or taurultam, with 2% of the taurolidine and/or taurultam, in solution and 2% as fine crystals suspended within the gel.

In preferred embodiments for treatment of bone cancers, such as osteosarcomas, tumorous materials may be removed from bone, forming a cavity in a bone, and gel granules in accordance with the present invention are filled into the bone cavity so as to kill any remaining tumor cells, and/or inhibit or at least partially prevent further growth of tumor cells. As indicated above, bone material and/or bone mineral can be mixed with the gel. In addition, aqueous methylol transfer agent such as taurolidine and/or taurultam solution may be administered to the patient systemically, e.g., by intravenous infusion, during a treatment period, which may include prior to the surgery, during the surgery, and/or following the surgery.

Effective dosage amounts for systemic administration of a methylol transfer agent in accordance with the present invention may comprise pharmaceutical dosage units within the range of about 0.1-1,000 mg/kg subject body weight, preferably 150-450 mg/kg per day, and most preferably 300-450 mg/kg per day. Alternatively, the dosages can be administered on a grams/day basis, from about 2-60 g/day. Preferred doses may be in the range of about 2.5-30 g/day taurolidine, 4-60 g/day taurultam, or a mixture thereof. Most preferred doses are in the range of about 10-20 g/day taurolidine, 20-40 g/day taurultam, or a mixture thereof.

Suitable formulations for injection or infusion may comprise an isotonic solution containing one or more solubilizing agents, e.g., polyols such as glucose, in order to provide solutions of increased taurolidine and/or taurultam concentration. Such solutions are described in EP 253662B1. The concentration of taurolidine and/or taurultam in such solutions may be in the range 1-60 g/liter.

Methylol transfer agents are generally poorly soluble in water. Thus, it is often required to administer relatively large volumes of aqueous solutions containing taurolidine and/or taurultam, for example 10g to 30g of taurolidine and/or taurultam. Preferred solutions for administration in accordance with the present invention contain about 0.5 - 3%, about 1 - 3%, about 2 - 3%, or about 2% by weight taurolidine and/or taurultam. It may be convenient to administer these compounds by infusion in view of the relatively large volumes concerned, conveniently at intervals throughout the day.

Administration, preferably by infusion, of the total additional daily dose of methylol transfer agent can be carried out at a consistent rate over 24 hours, or according to a more rapid infusion schedule of the dose in portions, with breaks between each portion of the dose, e.g. infusion of 250 ml of a 2% taurolidine and/or taurultam solution (5 g dose) over 2 hours, followed by a brief break of 4 hours, repeated over the course of a 24 hour infusion period to achieve a total daily dose of 20 g. Alternatively, 250 ml of a 2% taurolidine and/or taurultam solution may be infused over one hour, with a one hour break between dose portions, and repeated until the daily dose is achieved, such that the total daily dose is provided over the course of less than 24 hours (i.e., approximately half the day), with no infusion occurring during the remainder of the day.

In accordance with one embodiment, four bottles (250 ml each) of 2% taurolidine and/or taurultam solution are administered intravenously to patients with cancer, at a rate of 40 drops per minute, one bottle every six hours. The therapy cycle generally is an administration phase of daily infusions for one week, followed by a rest phase of two weeks. Total treatment generally is at least two such cycles. Efficacy of taurolidine and/or taurultam 2% solution administered intravenously has been found to be particularly good with 25-28 bottles of 250 ml taurolidine and/or taurultam 2% solution being instilled per cycle.

In accordance with a further embodiment of the invention, the administration phase comprises a daily regimen whereby 250 ml of taurolidine and/or taurultam 2% solution is administered over the course of 2 hours, followed by a four hour break, repeated over 24 hours to achieve the total daily systemic dose.

In accordance with a further embodiment of the invention, the administration phase comprises a daily regimen whereby 250 ml of 2% taurolidine and/or taurultam solution is infused over one hour, followed by a one-hour break, and repeated until the daily dose is achieved. If the total dose is 20 g (for example), this regimen would provide the daily dose with four 250 ml infusions of 2% taurolidine over a 7 hour time span. No infusion occurs for the remainder of the day.

In some embodiments, patients are subjected to dosing cycles having an administration phase of at least 3 continuous days, and up to about 8 continuous days, each administration phase being followed by a non-administration phase of about 1 day to about 4 weeks, e.g., 1-14 days, or even 3, 4 or more weeks, during which the methylol transfer agent is not administered to the patient. During each administration phase, the methylol transfer agent is administered each day. For example, administration phases of 3, 4, 5, 6, 7 and/or 8 days can be utilized, and non-administration phases of 1, 2, 3, 4, 5, 6, 7, 8, 9, 10, 11, 12, 13, and/or 14 days may be utilized. At least 2 dosing cycles are utilized, preferably 5-10 or more dosing cycles are utilized. For example, 2, 3, 4, 5, 6, 7, 8, 9, 10 or more sequential dosing cycles can be utilized. In one embodiment, 6 dosing cycles, each with administration phases of 5 days are utilized, with each administration phase separated by a non-administration phase of 2 days. During each day of administration, 250 ml of taurolidine and/or taurultam 2% solution may be intravenously administered to the patient 4 times daily.

In another embodiment, a non-administration phase may be 1, 2, 3, 4 or more weeks in length, e.g., about 2-4 weeks. For example, sequential dosing cycles may be administered having an administration phase of 3-8 continuous days, e.g., 7 days, with, for example, 250 ml taurolidine 2% solution infused 4 times daily, followed by a non-administration phase of 1, 2, 3, 4, or more weeks, e.g., 3 weeks. As in the previous embodiments, at least 2 dosing cycles are utilized, preferably 5-10 or more dosing cycles.

Fluid and electrolyte replacement may be administered in connection with intravenous taurolidine and/or taurultam therapy.

An amount of 250 ml of full electrolyte solution is preferably be given at the same time and with the same infusion speed parallel to the infusion with 250 ml taurolidine 2%. Electrolytes and blood count should be monitored twice per day, and the central vein pressure should be checked once daily.

If a hypernatraemia is observed, first, it should be determined whether dehydration is the cause. Diuretic agents should only be used if fluid is replaced at the same time and after dehydration was ruled out as the reason.

If desired, autologous bone material and/or bone marrow cells may be purged of cancer cells which may contaminate a sample. Autologous bone material and/or bone marrow cells are derived from a subject, or another mammalian donor using standard methods. The autologous bone material and/or cells are treated by contacting them with a methylol transfer agent such as taurolidine and/or taurultam *in vitro* to eliminate contaminating tumor cells. After washing the treated autologous bone material and/or cells, the autologous bone material and/or bone marrow cell preparation is administered to a mammalian recipient. Stem cells thus treated may reconstitute the immune system of the recipient. This can be done in conjunction with treatment with a gel including methylol transfer agent such as taurolidine and/or taurultam, and/or administration of systemic methylol transfer agent such as taurolidine and/or taurultam, as herein described.

The methylol transfer agent is administered alone or in combination with one or more additional antineoplastic agents. In one embodiment, the supplemental agent kills tumors cells by a mechanism other than apoptosis. For example, an antimetabolite, a purine or pyrimidine analogue, an alkylating agent, crosslinking agent (e.g., a platinum compound), and intercalating agent, and/or an antibiotic is administered in a combination therapy regimen. The supplemental drug is given before, after, or simultaneously with the methylol-containing agent. For example, the methylol transfer agent can be co-administered with a fluoro-pyrimidine, such as 5-fluoro-uracil (5-FU). Effective daily dosage amounts of a fluoro-pyrimidine may be in the range of about 0.1-1,000 mg per pharmaceutical dosage unit. Effective dosage amounts of 5-FU also may be in the range of about 100-5,000 mg/m² body surface area, preferably about 200-1,000 mg/m² body surface area, more preferably about 500-600 mg/m² body surface area. 5-FU typically is provided in 250 mg or 500 mg ampules for injection, or 250 mg capsules for oral administration.

The invention is further illustrated by the following examples, which are not intended to be limiting.

### EXAMPLE 1

Edible gelatin (125 g) was dispersed in 1% aqueous taurolidine (1250 ml) for about 10 minutes and subsequently warmed to 60°C. with stirring. Aqueous formaldehyde (36%; 12 ml) was added to the liquid gel with stirring. The mixture was further stirred at 60°C for 10-15 minutes and then poured into clean pre-heated polyvinylchloride tubes (diameter 14 mm). The tubes were cooled overnight and cut into 15 cm lengths and cut open. The transparent rods so obtained were then washed in a 1% taurolidine solution for about 4 hours in order to remove excess formaldehyde. The formaldehyde was quantitatively detected by gas chromatography (GC-WLD) and the washing was continued until no further free formaldehyde diffused into the wash water. The detection limit for free formaldehyde by this method was 0.003%.

A number of the rods were granulated in a Zyliss electric mincer, with sieve openings of 4.5 mm.

The rods as well as the granulate were then enclosed in a sealable polyethylene foil envelope backed with aluminum foil previously washed with 70% isopropanol. This may then be sealed in a second similar sterile envelope.

For the formation of the granulate, the gel mass can also be molded in a larger vessel, such as a crystallisation dish, and on cooling the mass can be washed as above with 1% taurolidine solution and subsequently granulated in the electric mincer.

### EXAMPLE 2

Gelatin rods were prepared according to the procedure of Example 1 but using 2% aqueous taurolidine in the formation of the gel and in the washing step and forming rods of diameter 10 mm and 15 mm.

### EXAMPLE 3

The procedure of Example 1 was repeated using, in place of gelatin, 125 g of a mixture of gelatin and tropocollagen in the ratio 2:1. The tropocollagen was derived from animal skin (calf skin) with a molecular weight of approximately 130,000.

### EXAMPLE 4

The procedure of Example 1 was repeated, using instead of the gelatin, a mixture of collagen fibres and gelatin in the weight ratio 1:3. The product was less transparent than that obtained using gelatin alone. The collagen fibres were added in a 10% suspension in water. A similar product was prepared using a mixture of collagen fibres and gelatin in the ratio 1:2, the overall concentration of gelatin being increased to 20% and the quantity of 36% aqueous formaldehyde being increased to 24 ml.

### EXAMPLE 5

The procedure of Example 1 was repeated, but the washing step was carried out with 2% aqueous taurolidine containing isotonic Ringer lactate solution (0.22% lactic acid, 0.6% NaCl, 0.4% KCl, 0.4% CaCl₂, 6H₂O; neutralised with NaOH to be orange to phenol red indicator; sterilised for 15 minutes at 120°C.)

After formulation of the gel into a granulate, this was mixed with an equal weight of freshly obtained spongiosa under sterile conditions.

### EXAMPLE 6

10 g edible gelatine was stirred for thirty minutes in 100 ml of 2% aqueous taurolidine solution containing 5% polyvinylpyrrolidone (PVP). The pH was adjusted to 7.0 with 25% aqueous NaOH. 1 g of a 36/37% formaldehyde aqueous solution was added and the mixture stirred for a further five minutes, after which time 25 g of dibasic calcium phosphate was added and stirring continued whilst the solution was allowed to cool.

The gel was left to stand for at least 4, preferably 4-7 days following which it was cut into pieces measuring about 2x3x3 cm and then washed using either (a) a solution of "Drainasept"/NaCl or (b) a solution of Ringer lactate plus 2% taurolidine in each case washing was carried out for four hours after which time the solution was changed and the gel washed in fresh solution for a further four hours.

The gel was finally washed with 2% taurolidine solution until isotonicity was achieved. The osmotic pressure of the gels was as follows:
before washing 200-400 mmol/kg
after washing 280-320 mmol/kg,
as measured by a 5100 vapour pressure osmometer supplied by Wescor Inc.

The gel was granulated in a mincing machine to about 1-2 mm in particle size. If desired the gels were placed in a homogeniser (e.g. "Homocenta") and ground very finely to permit of their being injected into the cavity via a suitable drain. Finally the granulated gel was placed in plastic containers previously washed with isopropanol.

### EXAMPLE 7

1000 ml of an aqueous solution comprising 2.55% taurolidine and 5% "Kollidon" K 17 PF (polyvinylpyrrolidone) and distilled water to 1000 ml was warmed to 60°C and 100 g edible gelatin (S.O.260) of 260-280 Bloom grams dissolved therein. The pH was adjusted to 7.0 with 25% NaOH. 7.5 g of a 35% aqueous formaldehyde solution ("Merck") was added and the mixture stirred for 15 minutes. The gel was allowed to stand for 7 days, after which it was washed for 9 hours in an equal weight of Ringer lactate solution containing 10% taurolidine followed by 60 hours in fresh solution. The gel was granulated as before.

### EXAMPLE 8

2.0 g taurolidine were dissolved at 60°C in 94 g distilled water and, after cooling to room temperature, 1.5 g of lactic acid (approx. 91%) were added and the pH adjusted to 7.0 with 25% aqueous sodium hydroxide. 0.024 g potassium chloride, 0.024 g calcium chloride hexahydrate and 0.33 g sodium chloride were added. The solution was heated to 60°C and 2 g edible gelatin dispersed therein. The pH was adjusted to 7.0 with 25% NaOH. 0.035 g of 100% formaldehyde were than added and the solution stirred until completely clear. The solution was poured into an infusion flask (250 ml) and autoclaved for 20 minutes at 120°C.

### EXAMPLE 9

4.0 g taurolidine were dissolved in 100 g distilled water and 10 g edible gelatin dispersed with stirring. The pH was adjusted to 7.0 with 25% NaOH. The solution was warmed to 60°C for 10-15 minutes and poured into pre-heated polyvinylchloride tubes (diameter 14 mm). The tubes were allowed to stand for several days and then cut open. The transparent rods so obtained were found to have a firmness similar to that of gels obtained by cross-linking using formaldehyde. A small quantity of taurinamide was detected, indicating methylol transfer by the taurolidine.

### EXAMPLE 10

Example 8 was repeated using 4.75 g taurolidine and 5 g PVP.

### EXAMPLE 11

Taurolidine was tested against 10 oestosarcoma cell lines at concentrations of 50, 100 and 200 uM taurolidine. The cell lines were SAOS-2 (HTB-85), U2OS (HTB-96), HOS, 143B, LM5, Hu09 WT, Hu09 H3, Hu09 L13, MG-63 and MG-63 M8. Taurolidine inhibited all osteosarcoma cell lines tested. Inhibition with Taurolidine resulted in a dose-dependent increase in apoptic cells, and apoptosis was caspase-dependent. Taurolidine possesses potent anti-neoplastic activity against osteosarcoma cell lines.

### EXAMPLE 12

### Treatment regimen

Orthopaedic patients were treated with Taurolin® (Geistlich AG) Gel (Taurolidine Fine Granulate 4%). A number of patients with osteosarcoma were treated by debridement to remove of the bone tumor, replenishment of the cavity with Taurolin®-Gel mixed with autologous spongiosa or Bio-Oss® (Geistlich AG) and multiple drainage. No recurrence of the osteosarcomas were observed.

### Concentration

The concentration of Taurolidine in the gel formulation was 4%, half dissolved in the watery liquid phase, half as fine crystals dispersed in the gel.

### Size of granules

Particle size of the Taurolin® Gel granules was approximately 1.5 - 2 mm (diameter).

## Claims

1. A gel containing a tumour growth-inhibiting methylol transfer agent for use in the treatment, at least partial prevention, inhibition or reduction of the growth of a bone tumour in a subject, said use comprising at least partially removing a bone tumour from a subject and contacting an area of bone adjacent to where the tumour was at least partially removed with said gel.

2. The gel of claim 1 wherein said gel is resorbable.

3. The gel of claim 1 wherein said gel is an aqueous gel comprising cross-linked fibrous protein.

4. The gel of claim 1 wherein said gel comprises gelatin, which may be optionally at least partially crosslinked.

5. The gel of claim 1 wherein said methylol transfer agent comprises taurolidine, taurultam, a mixture thereof, or an equilibrium thereof.

6. The gel of claim 5 wherein said methylol transfer agent is at a concentration within said gel of 0.5 - 5% by weight.

7. The gel of claim 6 wherein said concentration is 2 - 3% solubilized methylol transfer agent in said gel, and 1 - 2% crystalline methylol transfer agent in said gel.

8. The gel of claim 1 wherein said gel comprises granules having a size within a range of 1 - 10 mm.

9. The gel of claim 1 wherein said gel is in a form of a shaped solid.

10. The gel of claim 9 wherein said shaped solid is a rod.

11. The gel of claim 1 wherein said gel is mixed with autologous bone material, synthetic bone mineral, natural bone mineral or a combination thereof and the thus formed mixture is filled into a cavity within said bone

12. The gel of claim 1 further comprising bone marrow cells mixed with said gel prior to contacting said area.

13. The gel of claim 1 wherein said use comprises filling said gel into a cavity in said bone after at least partially removing said tumour, a drain tube is installed into the subject leading from the cavity to outside of the subject, and the cavity is surgically closed.

14. The gel of claim 13 wherein said use further comprises instilling additional said gel into said cavity through said drain tube.

15. The gel of claim 1 wherein said tumour is a sarcoma or an osteosarcoma.
